(19) Europäisches Patentamt
European Patent Office
Office européen des brevets

(11) **EP 2 600 269 A2**

(12) **EUROPEAN PATENT APPLICATION**

(43) Date of publication:
05.06.2013 Bulletin 2013/23

(51) Int Cl.:
*G06F 19/24* (2011.01)          *G06F 19/12* (2011.01)

(21) Application number: **12195130.5**

(22) Date of filing: **30.11.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **03.12.2011   US 201161566644 P**
**03.12.2011   US 201161566642 P**

(71) Applicant: **Medeolinx, LLC**
**Indianapolis, IN 46202 (US)**

(72) Inventors:
• **Yue Chen, Jake**
  **Indianapolis, IN 46280 (US)**
• **Wu, Xiaogang**
  **Indianapolis, IN 46202 (US)**

(74) Representative: **Decamps, Alain René François et al**
**Office Kirkpatrick S.A.**
**Avenue Wolfers, 32**
**1310 La Hulpe (BE)**

(54) **Microarray sampling and network modeling for drug toxicity prediction**

(57)     A computational systems pharmacology framework consisting of microarray sampling of gene expression and metabolic data, statistical modeling and machine learning based on comprehensive integration of systems biology data, including drug target data, protein-protein interaction (PPI) networks, and gene ontology (GO) annotations, and reported drug side effects, can predict drug toxicity or drug adverse reactions (ADRs). A disease-specific pathway model is first constructed with proteins and drugs important to the disease by using computational connectivity maps (C-Maps). Through the pathway model-based ranking algorithm, ideal drugs or optimized drug combination can be discovered for a patient to modulate the gene expression profile of this patient close to those in healthy individuals at pathway-level.

Figure 3

EP 2 600 269 A2

**Description**

Over 1500 Mendelian conditions whose molecular cause is unknown are listed in the Online Mendelian Inheritance in Man (OMIM) database. Additionally, almost all medical conditions are in some way influenced by human genetic variation. The identification of genes associated with these conditions is a goal of numerous research groups, in order to both improve medical care and better understand gene functions, interactions, and pathways. Sequencing large numbers of candidate genes remains a time-consuming and expensive task, and it is often not possible to identify the correct disease gene by inspection of the list of genes within the interval.

A number of computational approaches toward candidate-gene prioritization have been developed that are based on functional annotation, gene-expression data, or sequence-based features. High-throughput technologies have produced vast amounts of protein-protein interaction data, which represent a valuable resource for candidate-gene prioritization, because genes related to a specific or similar disease phenotype tend to be located in a specific neighborhood in the protein-protein interaction network. However, only relatively simple methods for exploring biological networks have been applied to the problem of candidate-gene prioritization, such as the search for direct neighbors of other disease genes and the calculation of the shortest path between candidates and known disease proteins.

SUMMARY

The invention relates to drug toxicity prediction based on network modeling and analysis combined with microarray sample data. More specifically, the present disclosure relates to computational methods, systems, devices, and/or apparatuses for predicting drug toxicity or drug adverse reaction (ADR) by using drug target-expanding protein-protein interaction (PPI) network modeling, and/or drug target-expanding gene ontology (GO) network modeling.

Recent research on drug side effects has drawn attention to the inadequacy of the traditional "one drug, one target, and causal effect" model. Modem drugs are designed to regulate the functions of specific target proteins, or "drug targets". Efficacious drugs can break through human barriers of absorption, discretion, metabolism, and excretion to achieve desirable "on-target" effects. However, drugs may also bind to "off-target" proteins, potentially leading to unwanted side effects, which range from mild drowsiness to deadly cardiotoxicity. More appropriate models must be developed to take advantage of complex molecular responses of drugs in cells, by exploiting fully the relationships between chemical compounds, protein targets, and side effects observed at the physiological level.

Systematic and quantitative investigation of adverse side effects has become increasingly important due to rising concerns about the cytotoxicity of drugs in development. Studies of drug toxicity and unintended side effects can lead to improved drug safety and efficacy. One promising strategy comes from molecular systems biology in the form of "systems pharmacology". Although the importance between systems biology and drug toxicity had been recognized, there had been no published report about how to practically predict drug toxicity by using biomolecular interaction and/or annotation information.

The present invention involves a computational systems pharmacology framework consisting of statistical modeling and machine learning to predict drug toxicity or drug adverse reaction (ADR). The computational framework is based on comprehensive integration of systems biology data, including drugs, protein targets, molecular annotation, and reported drug side effects. First, drug-target interactions are expanded in global human protein-protein interaction (PPI) networks to build drug target-expanding PPI networks. Second, drug targets are enriched by their gene ontology (GO) annotations to build drug target-expanding GO networks. Third, ADR information for each drug is combined with drug target-expanding PPI networks and/or drug target-expanding GO networks. Finally, statistical modeling and machine learning are applied to building the ADR classification/prediction model. Cross validation and feature selection are also used to train this drug toxicity prediction model.

In one embodiment, the present invention relates to a toxicity analysis tool comprising a patient analysis, database, network interaction, and toxicity models. The patient analysis module includes a microarray with a computer system and is configured to obtain gene expression information about a particular patient. The database module is configured to provide a set of targets for known interactions of a particular drug. The network interaction module is configured to expand said set of targets based on network interaction information to produce an expanded set of targets. The toxicity module is configured to determine if a toxicity reaction is likely based on said expanded set of targets, said toxicity module outputting an evaluation of the likelihood of toxicity for the particular drug with the particular patient. The patient analysis module is also configured to obtain metabolite information. The database module includes at least one of drug and drug target information and drug side effect information. The network interaction module uses a protein-protein interaction network model, and also uses gene ontology information including hierarchical terms, biological processes, cellular components, and molecular functions. The toxicity module includes a prediction model configured to execute at least one of support vector machine software and logistical regression analysis software. The extended set of targets includes feature information associated with each target, and the tool further includes a feature selection module configured to remove elements of the extended set of targets based on said feature information. The feature selection module is configured to filter said extended set of targets based on associated feature information having a p-value under a predetermined value, for example about 0.05. The tool further includes a cross-validation module configured to balance the extended set of targets, for example by partitioning the extended set of targets into a plurality of training sets and a testing set, and then balancing the plurality of training sets.

In another embodiment, the present invention relates to a method of determining toxicity. First is the step of obtaining gene expression information about a particular patient. Then, at least one database is accessed and a set of targets for known interactions of a particular drug are extracted. The set of targets is expanded based on network interaction information to produce an expanded set of targets. A toxicity reaction is determined to be likely based on the expanded set of targets, and an evaluation of the likelihood of toxicity for the particular drug is output. A further step of obtaining at least one of gene expression information and metabolite information of a particular patient may be performed, to evaluate toxicity based on the particular patient. The accessing step includes accessing at least one of drug and drug target information and drug side effect information. The expanding step uses a protein-protein interaction network model, and uses gene ontology information including hierarchical terms, biological processes, cellular components, and molecular functions. The determining step includes executing at least one of support vector machine software and logistical regression analysis software. The extended set of targets includes feature information associated with each target, and the method further includes removing elements of the extended set of targets based on feature information. The removing step includes filtering the extended set of targets based on associated feature information having a p-value under a predetermined value, for example about 0.05. The method further includes the step of cross-validation by balancing the extended set of targets, for example by partitioning the extended set of targets into a plurality of training sets and a testing set, and then balancing the plurality of training sets.

BRIEF DESCRIPTION OF THE DRAWINGS

The above mentioned and other features and obj ects of this invention, and the manner of attaining them, will become more apparent and the invention itself will be better understood by reference to the following description of an embodiment of the invention taken in conjunction with the accompanying drawings, wherein:

Figure 1 is a schematic diagrammatic view of a network system in which embodiments of the present invention may be utilized.

Figure 2 is a block diagram of a computing system (either a server or client, or both, as appropriate), with optional input devices (e.g., keyboard, mouse, touch screen, etc.) and output devices, hardware, network connections, one or more processors, and memory/storage for data and modules, etc. which may be utilized in conjunction with embodiments of the present invention.

Figure 3 is a schematic diagram illustrating a framework for drug toxicity or ADR prediction by using drug target-expanding PPI network modeling and/or drug target-expanding GO network modeling.

Figure 4A is a chart, Figure 4B is a network diagram, and Figure 4C is a flow diagram all illustrating drug target vs. drug side effect and an example of drug target-expanding network.

Figures 5A and 5B are graph diagrams illustrating the classification performance comparison for statistical modeling and machine learning by using different PPI confidence levels.

Figures 6A and 6B are graph diagrams illustrating the classification performance comparison for statistical modeling and machine learning by using different GO annotation levels.

Figure 7 is a network diagram illustrating the cardiotoxicity-associated PPI network built by using drug target-expanding PPI network modeling.

Figure 8 is a schematic diagram illustrating a framework for drug efficacy prediction by using an integrative pathway modeling approach and a ranking algorithm based on the integrative pathway models.

Figure 9 is a symbolic diagram illustrating the classification of a drug's pharmacological effect on targeted proteins.

Figure 10 is a multi-dimensional chart diagram illustrating the heat map for a simple network shown in the left of the figure. Columns represent the edges while rows represent nodes. '1' indicates activation or over-expression while '-1' indicates inhibition or under-expression.

Figure 11 is a graph diagram illustrating a breast cancer drug list with PET scores by applying the PET algorithm to two gene expression profiles (GSE10866 and GSE3193) with the PEN model. For each drug, there are two PET scores, displayed as two bars. The upper one is from GSE10866, and the lower one is from GSE3193.

Figure 12 is a graph diagram illustrating a breast cancer drug list with PET scores by applying the PET algorithm to two gene expression profiles (GSE10866 and GSE3193) without the PEN model. For each drug, there are two PET scores, displayed as two bars. The upper one is from GSE10866, and the lower one is from GSE3193.

Corresponding reference characters indicate corresponding parts throughout the several views. Although the drawings represent embodiments of the present invention, the drawings are not necessarily to scale and certain features may be exaggerated in order to better illustrate and explain the present invention. The flow charts and screen shots are also representative in nature, and actual embodiments of the invention may include further features or steps not shown in the drawings. The exemplification set out herein illustrates an embodiment of the invention, in one form, and such exemplifications are not to be construed as limiting the scope of the invention in any manner.

<u>DETAILED DESCRIPTION</u>

The embodiment disclosed below is not intended to be exhaustive or limit the invention to the precise form disclosed in the following detailed description. Rather, the embodiment is chosen and described so that others skilled in the art may utilize its teachings.

In the field of molecular biology, gene expression profiling is the measurement of the activity (the expression) of thousands of genes at once, to create a global picture of cellular function including protein and other cellular building blocks. These profiles may, for example, distinguish between cells that are actively dividing or otherwise reacting to the current bodily condition, or show how the cells react to a particular treatment such as positive drug reactions or toxicity reactions. Many experiments of this sort measure an entire genome simultaneously, that is, every gene present in a particular cell, as well as other important cellular building blocks.

DNA Microarray technology measures the relative activity of previously identified target genes. Sequence based techniques, like serial analysis of gene expression (SAGE, SuperSAGE) are also used for gene expression profiling. SuperSAGE is especially accurate and may measure any active gene, not just a predefined set. The advent of next-generation sequencing has made sequence based expression analysis an increasingly popular, "digital" alternative to microarrays called RNA-Seq.

Expression profiling provides a view to what a patient's genetic materials are actually doing at a point in time. Genes contain the instructions for making messenger RNA (mRNA), but at any moment each cell makes mRNA from only a fraction of the genes it carries. If a gene is used to produce mRNA, it is considered "on", otherwise "off". Many factors determine whether a gene is on or off, such as the time of day, whether or not the cell is actively dividing, its local environment, and chemical signals from other cells. For instance, skin cells, liver cells and nerve cells turn on (express) somewhat different genes and that is in large part what makes them different. Therefore, an expression profile allows one to deduce a cell's type, state, environment, and so forth.

Expression profiling experiments often involve measuring the relative amount of mRNA expressed in two or more experimental conditions. For example, genetic databases have been created that reflect a normative state of a healthy patient, which may be contrasted with databases that have been created from a set of patient's with a particular disease or other condition. This contrast is relavent because altered levels of a specific sequence of mRNA suggest a changed need for the protein coded for by the mRNA, perhaps indicating a homeostatic response or a pathological condition. For example, higher levels of mRNA coding for one particular disease is indicative that the cells or tissues under study are responding to the effects of the particular disease. Similarly, if certain cells, for example a type of cancer cells, express higher levels of mRNA associated with a particular transmembrane receptor than normal cells do, the expression of that receptor is indicative of cancer. A drug that interferes with this receptor may prevent or treat that type of cancer. In developing a drug, gene expression profiling may assess a particular drug's toxicity, for example by detecting changing levels in the expression of certain genes that constitute a biomarker of drug metabolism.

For a type of cell, the group of genes and other cellular materials whose combined expression pattern is uniquely characteristic to a given condition or disease constitutes the gene signature of this condition or disease. Ideally, the gene signature is used to detect a specific state of a condition or disease to facilitates selection of treatments. Gene Set Enrichment Analysis (GSEA) and similar methods take advantage of this kind of logic and uses more sophisticated statistics. Component genes in real processes display more complex behavior than simply expressing as a group, and the amount and variety of gene expression is meaningful. In any case, these statistics measure how different the behavior of some small set of genes is compared to genes not in that small set.

One way to analyze sets of genes and other cellular materials apparent in gene expression measurement is through the use of pathway models and network models. Many protein-protein interactions (PPIs) in a cell form protein interaction networks (PINs) where proteins are nodes and their interactions are edges. There are dozens of PPI detection methods to identify such interactions. In addition, gene regulatory networks (DNA-protein interaction networks) model the activity of genes which is regulated by transcription factors, proteins that typically bind to DNA. Most transcription factors bind to multiple binding sites in a genome. As a result, all cells have complex gene regulatory networks which may be combined with PPIs to link together these various connections. The chemical compounds of a living cell are connected by biochemical reactions which convert one compound into another. The reactions are catalyzed by enzymes. Thus, all compounds in a cell are parts of an intricate biochemical network of reactions which is called the metabolic network, which may further enhance PPI and/or DNA-protein network models. Further, signals are transduced within cells or in between cells and thus form complex signaling networks that may further augment such genetic interaction networks. For instance, in the MAPK/ERK pathway is transduced from the cell surface to the cell nucleus by a series of protein-protein interactions, phosphorylation reactions, and other events. Signaling networks typically integrate protein-protein interaction networks, gene regulatory networks, and metabolic networks.

The detailed descriptions which follow are presented in part in terms of algorithms and symbolic representations of operations on data bits within a computer memory representing genetic profiling information derived from patient sample data and populated into network models. A computer generally includes a processor for executing instructions and memory for storing instructions and data. When a general purpose computer has a series of machine encoded instructions stored in its memory, the computer operating on such encoded instructions may become a specific type of machine, namely a computer particularly configured to perform the operations embodied by the series of instructions. Some of the instructions may be adapted to produce signals that control operation of other machines and thus may operate through those control signals to transform materials far removed from the computer itself. These descriptions and representations are the means used by those skilled in the art of data processing arts to most effectively convey the substance of their work to others skilled in the art.

An algorithm is here, and generally, conceived to be a self-consistent sequence of steps leading to a desired result. These steps are those requiring physical manipulations of physical quantities. Usually, though not necessarily, these quantities take the form of electrical or magnetic pulses or signals capable of being stored, transferred, transformed, combined, compared, and otherwise manipulated. It proves convenient at times, principally for reasons of common usage, to refer to these signals as bits, values, symbols, characters, display data, terms, numbers, or the like as a reference to the physical items or manifestations in which such signals are embodied or expressed. It should be borne in mind, however, that all of these and similar terms are to be associated with the appropriate physical quantities and are merely used here as convenient labels applied to these quantities.

Some algorithms may use data structures for both inputting information and producing the desired result. Data structures greatly facilitate data management by data processing systems, and are not accessible except through sophisticated software systems. Data structures are not the information content of a memory, rather they represent specific electronic structural elements which impart or manifest a physical organization on the information stored in memory. More than mere abstraction, the data structures are specific electrical or magnetic structural elements in memory which simultaneously represent complex data accurately, often data modeling physical characteristics of related items, and provide increased efficiency in computer operation.

Further, the manipulations performed are often referred to in terms, such as comparing or adding, commonly associated with mental operations performed by a human operator. No such capability of a human operator is necessary, or desirable in most cases, in any of the operations described herein which form part of the present invention; the operations are machine operations. Useful machines for performing the operations of the present invention include general purpose digital computers or other similar devices. In all cases the distinction between the method operations in operating a computer and the method of computation itself should be recognized. The present invention relates to a method and apparatus for operating a computer in processing electrical or other (e.g., mechanical, chemical) physical signals to generate other desired physical manifestations or signals. The computer operates on software modules, which are collections of signals stored on a media that represents a series of machine instructions that enable the computer processor to perform the machine instructions that implement the algorithmic steps. Such machine instructions may be the actual computer code the processor interprets to implement the instructions, or alternatively may be a higher level coding of the instructions that is interpreted to obtain the actual computer code. The software module may also include a hardware component, wherein some aspects of the algorithm are performed by the circuitry itself rather as a result of an instruction.

The present invention also relates to an apparatus for performing these operations. This apparatus may be specifically constructed for the required purposes or it may comprise a general purpose computer as selectively activated or reconfigured by a computer program stored in the computer. The algorithms presented herein are not inherently related to any particular computer or other apparatus unless explicitly indicated as requiring particular hardware. In some cases, the computer programs may communicate or relate to other programs or equipments through signals configured to particular protocols which may or may not require specific hardware or programming to interact. In particular, various general purpose machines may be used with programs written in accordance with the teachings herein, or it may prove more convenient to construct more specialized apparatus to perform the required method steps. The required structure for a variety of these machines will appear from the description below.

The present invention may deal with "object-oriented" software, and particularly with an "object-oriented" operating system. The "object-oriented" software is organized into "objects", each comprising a block of computer instructions describing various procedures ("methods") to be performed in response to "messages" sent to the object or "events" which occur with the object. Such operations include, for example, the manipulation of variables, the activation of an object by an external event, and the transmission of one or more messages to other objects.

Messages are sent and received between objects having certain functions and knowledge to carry out processes. Messages are generated in response to user instructions, for example, by a user activating an icon with a "mouse" pointer generating an event. Also, messages may be generated by an obj ect in response to the receipt of a message. When one of the objects receives a message, the object carries out an operation (a message procedure) corresponding to the message and, if necessary, returns a result of the operation. Each object has a region where internal states (instance variables) of the object itself are stored and where the other objects are not allowed to access. One feature of the object-oriented system is inheritance. For example, an object for drawing a "circle" on a display may inherit functions and knowledge from another object for drawing a "shape" on a display.

A programmer "programs" in an object-oriented programming language by writing individual blocks of code each of which creates an object by defining its methods. A collection of such obj ects adapted to communicate with one another by means of messages comprises an object-oriented program. Object-oriented computer programming facilitates the modeling of interactive systems in that each component of the system can be modeled with an obj ect, the behavior of each component being simulated by the methods of its corresponding object, and the interactions between components being simulated by messages transmitted between objects.

An operator may stimulate a collection of interrelated objects comprising an object-oriented program by sending a message to one of the objects. The receipt of the message may cause the object to respond by carrying out predetermined functions which may include sending additional messages to one or more other objects. The other objects may in turn carry out additional functions in response to the messages they receive, including sending still more messages. In this manner, sequences of message and response may continue indefinitely or may come to an end when all messages have been responded to and no new messages are being sent. When modeling systems utilizing an object-oriented language, a programmer need only think in terms of how each component of a modeled system responds to a stimulus and not in terms of the sequence of operations to be performed in response to some stimulus. Such sequence of operations naturally flows out of the interactions between the objects in response to the stimulus and need not be preordained by the programmer.

Although object-oriented programming makes simulation of systems of interrelated components more intuitive, the operation of an object-oriented program is often difficult to understand because the sequence of operations carried out by an object-oriented program is usually not immediately apparent from a software listing as in the case for sequentially organized programs. Nor is it easy to determine how an object-oriented program works through observation of the readily apparent manifestations of its operation. Most of the operations carried out by a computer in response to a program are "invisible" to an observer since only a relatively few steps in a program typically produce an observable computer output.

In the following description, several terms which are used frequently have specialized meanings in the present context. The term "object" relates to a set of computer instructions and associated data which can be activated directly or indirectly by the user. The terms "windowing environment", "running in windows", and "object oriented operating system" are used to denote a computer user interface in which information is manipulated and displayed on a video display such as within bounded regions on a raster scanned video display. The terms "network", "local area network", "LAN", "wide area network", or "WAN" mean two or more computers which are connected in such a manner that messages may be transmitted between the computers. In such computer networks, typically one or more computers operate as a "server", a computer with large storage devices such as hard disk drives and communication hardware to operate peripheral devices such as printers or modems. Other computers, termed "workstations", provide a user interface so that users of computer networks can access the network resources, such as shared data files, common peripheral devices, and inter-workstation communication. Users activate computer programs or network resources to create "processes" which include both the general operation of the computer program along with specific operating characteristics determined by input variables and its environment. Similar to a process is an agent (sometimes called an intelligent agent), which is a process that gathers information or performs some other service without user intervention and on some regular schedule. Typically, an agent, using parameters typically provided by the user, searches locations either on the host machine or at some other point on a network, gathers the information relevant to the purpose of the agent, and presents it to the user on a periodic basis. A "module" refers to a portion of a computer system and/or software program that carries out one or more specific functions and may be used alone or combined with other modules of the same system or program.

The term "desktop" means a specific user interface which presents a menu or display of objects with associated settings for the user associated with the desktop. When the desktop accesses a network resource, which typically requires an application program to execute on the remote server, the desktop calls an Application Program Interface, or "API", to allow the user to provide commands to the network resource and observe any output. The term "Browser" refers to a program which is not necessarily apparent to the user, but which is responsible for transmitting messages between the desktop and the network server and for displaying and interacting with the network user. Browsers are designed to utilize a communications protocol for transmission of text and graphic information over a world wide network of computers, namely the "World Wide Web" or simply the "Web". Examples of Browsers compatible with the present invention include the Internet Explorer program sold by Microsoft Corporation (Internet Explorer is a trademark of Microsoft Corporation), the Opera Browser program created by Opera Software ASA, or the Firefox browser program distributed by the Mozilla Foundation (Firefox is a registered trademark of the Mozilla Foundation). Although the following description details such operations in terms of a graphic user interface of a Browser, the present invention may be practiced with text based interfaces, or even with voice or visually activated interfaces, that have many of the functions of a graphic based Browser.

Browsers display information which is formatted in a Standard Generalized Markup Language ("SGML") or a HyperText Markup Language ("HTML"), both being scripting languages which embed non-visual codes in a text document through the use of special ASCII text codes. Files in these formats may be easily transmitted across computer networks, including global information networks like the Internet, and allow the Browsers to display text, images, and play audio and video recordings. The Web utilizes these data file formats to conjunction with its communication protocol to transmit such information between servers and workstations. Browsers may also be programmed to display information provided in an eXtensible Markup Language ("XML") file, with XML files being capable of use with several Document Type Definitions ("DTD") and thus more general in nature than SGML or HTML. The XML file may be analogized to an object, as the data and the stylesheet formatting are separately contained (formatting may be thought of as methods of displaying information, thus an XML file has data and an associated method).

The terms "personal digital assistant" or "PDA", as defined above, means any handheld, mobile device that combines computing, telephone, fax, e-mail and networking features. The terms "wireless wide area network" or "WWAN" mean a wireless network that serves as the medium for the transmission of data between a handheld device and a computer. The term "synchronization" means the exchanging of information between a first device, e.g. a handheld device, and a second device, e.g. a desktop computer, either via wires or wirelessly. Synchronization ensures that the data on both devices are identical (at least at the time of synchronization).

In wireless wide area networks, communication primarily occurs through the transmission of radio signals over analog, digital cellular or personal communications service ("PCS") networks. Signals may also be transmitted through microwaves and other electromagnetic waves. At the present time, most wireless data communication takes place across cellular systems using second generation technology such as code-division multiple access ("CDMA"), time division multiple access ("TDMA"), the Global System for Mobile Communications ("GSM"), Third Generation (wideband or "3G"), Fourth Generation (broadband or "4G"), personal digital cellular ("PDC"), or through packet-data technology over analog systems such as cellular digital packet data (CDPD") used on the Advance Mobile Phone Service ("AMPS").

The terms "wireless application protocol" or "WAP" mean a universal specification to facilitate the delivery and presentation of web-based data on handheld and mobile devices with small user interfaces. "Mobile Software" refers to the software operating system which allows for application programs to be implemented on a mobile device such as a mobile telephone or PDA. Examples of Mobile Software are Java and Java ME (Java and JavaME are trademarks of Sun Microsystems, Inc. of Santa Clara, California), BREW (BREW is a registered trademark of Qualcomm Incorporated of San Diego, California), Windows Mobile (Windows is a registered trademark of Microsoft Corporation of Redmond, Washington), Palm OS (Palm is a registered trademark of Palm, Inc. of Sunnyvale, California), Symbian OS (Symbian is a registered trademark of Symbian Software Limited Corporation of London, United Kingdom), ANDROID OS (ANDROID is a registered trademark of Google, Inc. of Mountain View, California), and iPhone OS (iPhone is a registered trademark of Apple, Inc. of Cupertino, California), and Windows Phone 7. "Mobile Apps" refers to software programs written for execution with Mobile Software.

"PACS" refers to Picture Archiving and Communication System (PACS) involving medical imaging technology for storage of, and convenient access to, images from multiple source machine types. Electronic images and reports are transmitted digitally via PACS; this eliminates the need to manually file, retrieve, or transport film jackets. The universal format for PACS image storage and transfer is DICOM (Digital Imaging and Communications in Medicine). Non-image data, such as scanned documents, may be incorporated using consumer industry standard formats like PDF (Portable Document Format), once encapsulated in DICOM. A PACS typically consists of four major components: imaging modalities such as X-ray computed tomography (CT) and magnetic resonance imaging (MRI) (although other modalities such as ultrasound (US), positron emission tomography (PET), endoscopy (ES), mammograms (MG), Digital radiography (DR), computed radiography (CR), etc. may be included), a secured network for the transmission of patient information, workstations and mobile devices for interpreting and reviewing images, and archives for the storage and retrieval of images and reports. When used in a more generic sense, PACS may refer to any image storage and retrieval system.

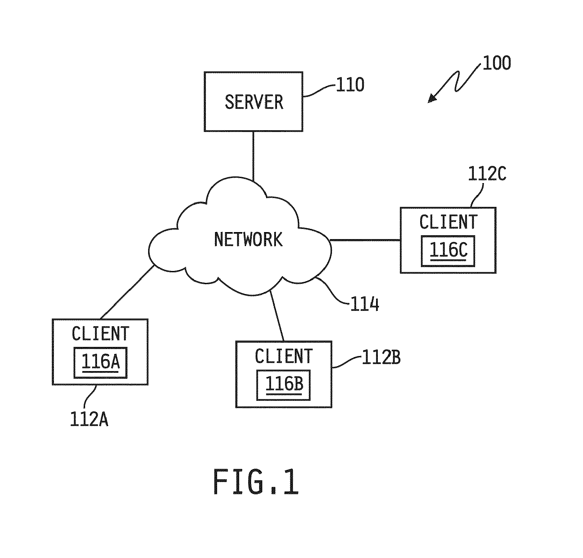

Figure 1 is a high-level block diagram of a computing environment 100 according to one embodiment. Figure 1 illustrates server 110 and three clients 112 connected by network 114. Only three clients 112 are shown in Figure 1 in order to simplify and clarify the description. Embodiments of the computing environment 100 may have thousands or millions of clients 112 connected to network 114, for example the Internet. Users (not shown) may operate software 116 on one of clients 112 to both send and receive messages network 114 via server 110 and its associated communications equipment and software (not shown).

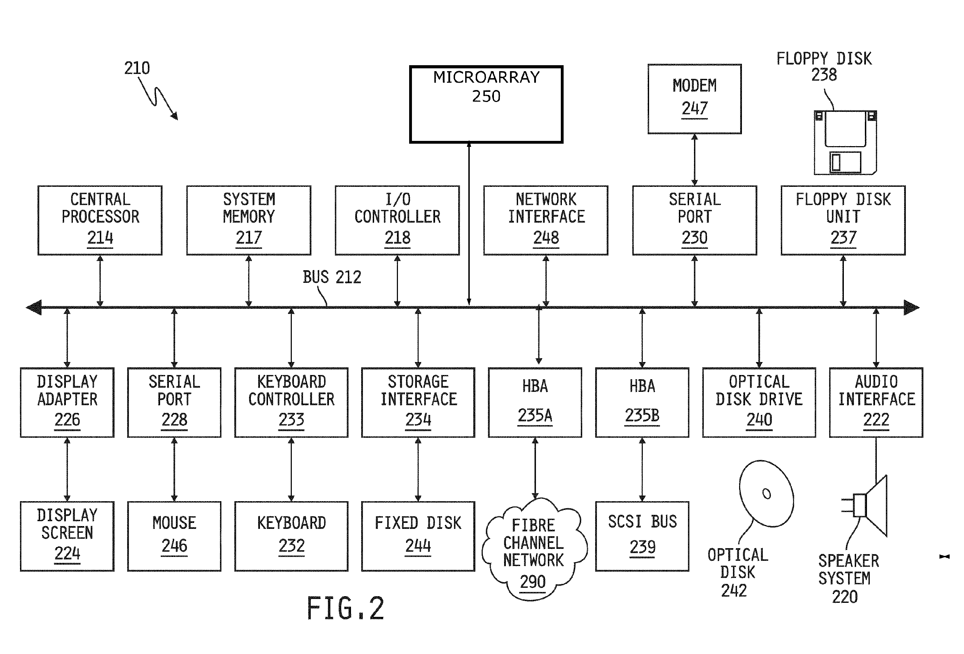

Figure 2 depicts a block diagram of computer system 210 suitable for implementing server 110 or client 112. Computer system 210 includes bus 212 which interconnects major subsystems of computer system 210, such as central processor 214, system memory 217 (typically RAM, but which may also include ROM, flash RAM, or the like), input/output controller 218, external audio device, such as speaker system 220 via audio output interface 222, external device, such as display screen 224 via display adapter 226, serial ports 228 and 230, keyboard 232 (interfaced with keyboard controller 233), storage interface 234, disk drive 237 operative to receive floppy disk 238, host bus adapter (HBA) interface card 235A operative to connect with Fibre Channel network 290, host bus adapter (HBA) interface card 235B operative to connect to SCSI bus 239, and optical disk drive 240 operative to receive optical disk 242. Also included are mouse 246 (or other point-and-click device, coupled to bus 212 via serial port 228), modem 247 (coupled to bus 212 via serial port 230), and network interface 248 (coupled directly to bus 212).

Bus 212 allows data communication between central processor 214 and system memory 217, which may include read-only memory (ROM) or flash memory (neither shown), and random access memory (RAM) (not shown), as previously noted. RAM is generally the main memory into which operating system and application programs are loaded. ROM or flash memory may contain, among other software code, Basic Input-Output system (BIOS) which controls basic hardware operation such as interaction with peripheral components. Applications resident with computer system 210 are generally stored on and accessed via computer readable media, such as hard disk drives (e.g., fixed disk 244), optical drives (e.g., optical drive 240), floppy disk unit 237, or other storage medium. Additionally, applications may be in the form of electronic signals modulated in accordance with the application and data communication technology when accessed via network modem 247 or interface 248 or other telecommunications equipment (not shown).

Storage interface 234, as with other storage interfaces of computer system 210, may connect to standard computer readable media for storage and/or retrieval of information, such as fixed disk drive 244. Fixed disk drive 244 may be part of computer system 210 or may be separate and accessed through other interface systems. Modem 247 may provide direct connection to remote servers via telephone link or the Internet via an internet service provider (ISP) (not shown). Network interface 248 may provide direct connection to remote servers via direct network link to the Internet via a POP (point of presence). Network interface 248 may provide such connection using wireless techniques, including digital cellular telephone connection, Cellular Digital Packet Data (CDPD) connection, digital satellite data connection or the like.

Many other devices or subsystems (not shown) may be connected in a similar manner (e.g., document scanners, digital cameras and so on). Conversely, all of the devices shown in Figure 2 need not be present to practice the present disclosure. Devices and subsystems may be interconnected in different ways from that shown in Figure 2. Operation of a computer system such as that shown in Fig. 2 is readily known in the art and is not discussed in detail in this application. Software source and/or object codes to implement the present disclosure may be stored in computer-readable storage media such as one or more of system memory 217, fixed disk 244, optical disk 242, or floppy disk 238. The operating system provided on computer system 210 may be a variety or version of either MS-DOS® (MS-DOS is a registered trademark of Microsoft Corporation of Redmond, Washington), WINDOWS® (WINDOWS is a registered trademark of Microsoft Corporation of Redmond, Washington), OS/2® (OS/2 is a registered trademark of International Business Machines Corporation of Armonk, New York), UNIX® (UNIX is a registered trademark of X/Open Company Limited of Reading, United Kingdom), Linux® (Linux is a registered trademark of Linus Torvalds of Portland, Oregon), or other known or developed operating system. In some embodiments, computer system 210 may take the form of a tablet computer, typically in the form of a large display screen operated by touching the screen. In tablet computer alternative embodiments, the operating system may be iOS® (iOS is a registered trademark of Cisco Systems, Inc. of San Jose, California, used under license by Apple Corporation of Cupertino, California), Android® (Android is a trademark of Google Inc. of Mountain View, California), Blackberry® Tablet OS (Blackberry is a registered trademark of Research In Motion of Waterloo, Ontario, Canada), webOS (webOS is a trademark of Hewlett-Packard Development Company, L.P. of Texas), and/or other suitable tablet operating systems.

Moreover, regarding the signals described herein, those skilled in the art recognize that a signal may be directly transmitted from a first block to a second block, or a signal may be modified (e.g., amplified, attenuated, delayed, latched, buffered, inverted, filtered, or otherwise modified) between blocks. Although the signals of the above described embodiments are characterized as transmitted from one block to the next, other embodiments of the present disclosure may include modified signals in place of such directly transmitted signals as long as the informational and/or functional aspect of the signal is transmitted between blocks. To some extent, a signal input at a second block may be conceptualized as a second signal derived from a first signal output from a first block due to physical limitations of the circuitry involved (e.g., there will inevitably be some attenuation and delay). Therefore, as used herein, a second signal derived from a first signal includes the first signal or any modifications to the first signal, whether due to circuit limitations or due to passage through other circuit elements which do not change the informational and/or final functional aspect of the first signal.

One peripheral device particularly useful with embodiments of the present invention is microarray 250. Generally, microarray 250 represents one or more devices capable of analyzing and providing genetic expression and other molecular information from patients. Microarrays may be manufactured in different ways, depending on the number of probes under examination, costs, customization requirements, and the type of analysis contemplated. Such arrays may have as few as 10 probes or over a million micrometre-scale probes, and are generally available from multiple commercial vendors. Each probe in a particular array is responsive to one or more genes, gene-expressions, proteins, enzymes, metabolites and/or other molecular materials, collectively referred to hereinafter as targets or target products.

In some embodiments, gene expression values from microarray experiments may be represented as heat maps to visualize the result of data analysis. In other embodiments, the gene expression values are mapped into a network structure and compared to other network structures, e.g. normalized samples and/or samples of patients with a particular condition or disease. In either circumstance, a simple patient sample may be analyzed and compared multiple times to focus or differentiate diagnoses or treatments. Thus, a patient having signs of multiple conditions or diseases may have microarray sample data analyzed several times to clarify possible diagnoses or treatments.

It is also possible, in several embodiments, to have multiple types of microarrays, each type having sensitivity to particular expressions and/or other molecular materials, and thus particularized for a predetermined set of targets. This allows for an iterative process of patient sampling, analysis, and further sampling and analysis to refine and personalize diagnoses and treatments for individuals. While each commercial vendor may have particular platforms and data formats, most if not all may be reduced to standardized formats. Further, sample data may be subject to statistical treatment for analysis and/or accuracy and precision so that individual patient data is a relevant as possible. Such individual data may be compared to large databases having thousands or millions sets of comparative data to assist in the experiment, and several such databases are available in data warehouses and available to the public. Due to the biological complexity of gene expression, the considerations of experimental design are necessary so that statistically and biologically valid conclusions may be drawn from the data.

Microarray data sets are commonly very large, and analytical precision is influenced by a number of variables. Statistical challenges include taking into account effects of background noise and appropriate normalization of the data. Normalization methods may be suited to specific platforms and, in the case of commercial platforms, some analysis may be proprietary. The relation between a probe and the mRNA that it is expected to detect is not trivial. Some mRNAs may cross-hybridize probes in the array that are supposed to detect another mRNA. In addition, mRNAs may experience amplification bias that is sequence or molecule-specific. Thirdly, probes that are designed to detect the mRNA of a particular gene may be relying on genomic Expression Sequence Tag (EST) information that is incorrectly associated with that gene.

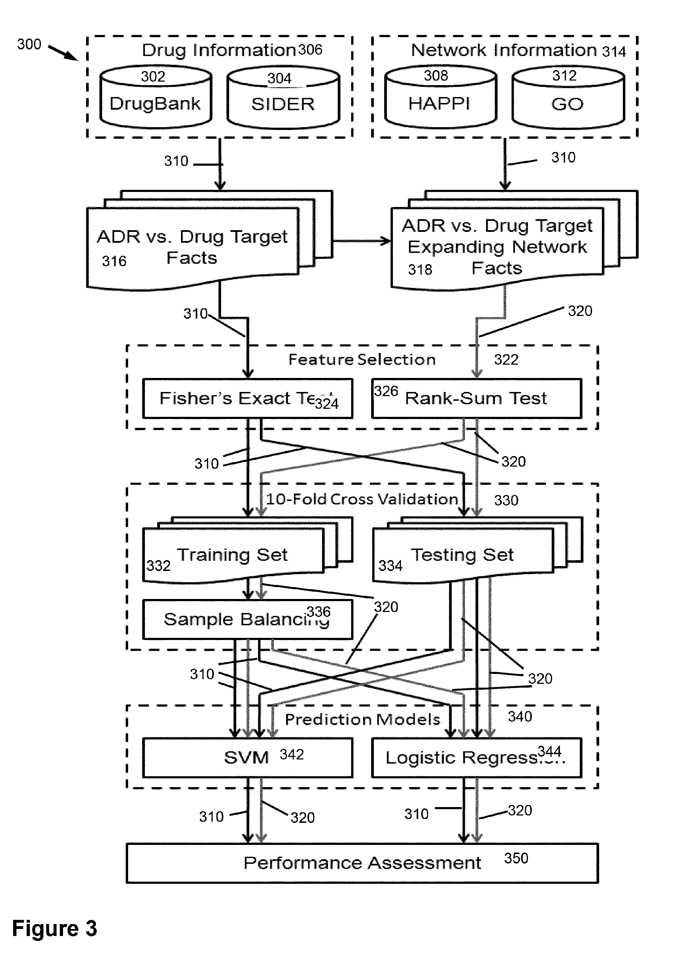

**Toxicity Analysis.** Framework 300 for predicting drug toxicity or drug adverse reaction (ADR) by using drug target-expanding protein-protein interaction (PPI) network modeling, and/or drug target-expanding gene ontology (GO) network modeling is shown in Figure 3, which includes drug and network information retrieval, feature selection, cross validation, sample balancing, prediction models, and performance assessment.

There are two types of data flows in the framework: 1) Arrows 310 indicate data flows for ADR vs. drug target facts. 2) Arrows 320 indicate data flows for ADR vs. drug target-expanding network facts, which are generated by integrating ADR vs. drug target facts with PPI and/or GO network information.

**1. Drug and network information retrieval:** First, DrugBank database 302 is exploited as a bioinformatics and chemoinformatics resource, which contains drug and drug target information. Up to May 2011, there were 5,461 drugs and 3,880 proteins, which formed 13,457 unique drug-target pairs in DrugBank 302, and they were extracted as main drug target information. A database module running on computer system 210 may serve as a computing mechanism to provide a set of targets for known interactions of a particular drug.

Second, the Side Effect Resource (SIDER) database 304 is also involved. This database aggregates FDA drug labels and disperses public information on ADRs. There were 877 drugs, 1,447 kinds of ADR, and 61,824 relationships among drugs and ADRs obtained from COSTART and Euphoria-related ADRs in SIDER. There are 578 drugs overlapped between DrugBank 302 and SIDER 304. Other relevant databases may also be included in Drug Information 306, including but not limited to the comprehensive drug information provided through drugs.com, drug target information from the Manually Annotated Targets and Drugs Online Resource (MATADOR at http://matador.embl.de/), and adverse drug effect information from the FDA's Adverse Event Reporting System (formerly AERS, now FAERS), and other databases having similar information.

Third, the Human Annotated and Predicted Protein Interactions (HAPPI) database 308 may be used as a global human PPI resource, and optionally a patient microarray sample (for example, obtained by use of microarray 250 as part of a patient module running on computer 210) may also be included in network information 314. HAPPI 308 integrates the Human Protein Reference Database (HPRD), the Biomolecular Interaction Network Database (BIND), the Molecular INTeraction database (MINT), the Search Tool for the Retrieval of Interactive Genes (STRING), and the Online Predicted Human Interaction Database (OPHID). Most importantly, HAPPI 308 provides a confidence star quality rating from 1 to 5 for each interaction based on the initial data sources, data generation methods, and number of literature references for the interaction. Excluding self PPIs, there are 116,275 PPIs, 61,698 PPIs, 48,481 PPIs, 24,750 PPIs, and 35,752 PPIs involved in the data set from 1 star to 5 stars, respectively. This data may be used to expand the network of drug targets.

Finally, Gene Ontology (GO) project 312 provides hierarchical terms, including biological processes, cellular components, and molecular functions, to describe the characteristics and annotations of gene product. Here we only use biological processes, from a general term "biological process" in level 1 to specific terms in level 15, to expand the features in the prediction models from drug targets to the GO terms in order to investigate the biological meanings between drug targets and ADRs. There are 3,715 biological process terms utilized for annotating the drug targets. Other databases involving interactions of metabolites, RNA, DNA, proteins, other gene expression information and other macromolecules may be included in Network Information 314, including but not limited to Anatomical Therapeutic Chemical (ATC) Classification System, which divides drugs into different groups according to the organ or system on which they act and/or their therapeutic and chemical characteristics, and other databases having similar information.

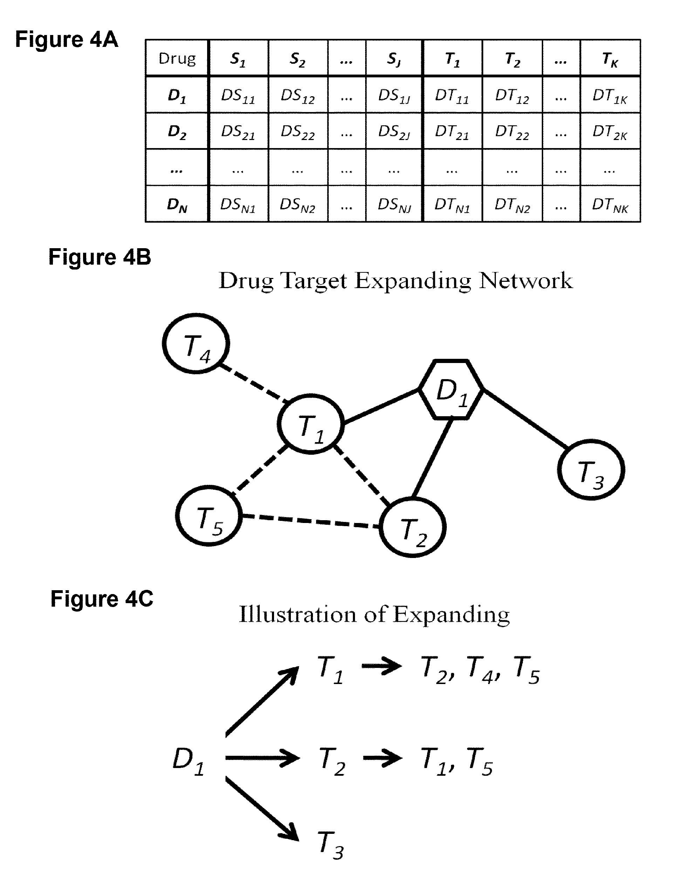

**2. ADR vs. drug target/drug target-expanding network facts:** By combining the drug target information in DrugBank 302 with the ADR information in SIDER 304, we obtained tabulation 306 of ADR vs. drug target facts. The facts follow the format shown in Figure 4A. If drug n has a side effect j, the value in cell $DS_{nj}$ ($n = 1...N$, and $j = 1...J$) at the intersection of column $S_j$ and row $D_n$ is 1 or "TRUE"; otherwise, it is 0 or "FALSE". So does the value in cell $DT_{nk}$ ($n = 1...N$, and $k = 1...K$) at the intersection of column $T_k$ and row $D_n$ if drug $n$ docks to drug target $k$. The binary data $DS_{nj}$ and $DT_{nk}$, representing the ADR vs. drug target facts, may be then used for prediction model training and testing: each ADR $S_j$ is prediction output (response variable) and targets from $T_1$ to $T_K$ are features (dependent variables).

When the drug targets expand one level in a PPI network or are annotated by using the GO terms, the value in cell $DT_{nk}$ will be integer instead of binary, because the association between drug n and drug target k could be repeatedly present in drug target expanding network. Figure 4B shows an example of a drug target-expanding network, and Figure 4C shows the drug target-expanding process and the repeated presences of $T_1$, $T_2$, and $T_5$. The repeat number here can be regarded as the weight of the relationship between drug and target under network level. In this way, software executing according to the tabulation of 306 on computer system 210 may serve as a network interaction module that is configured to expand a set of targets based on network information 314 to produce an expanded set of targets.

**3. Feature selection:** Since thousands of features (drug targets) are required to build prediction models, Feature Selection process 322 may be exhaustive and memory consuming. Moreover, some statistics tools, such as R, have memory limitations. Hence, such limitations may be mitigated against by filtering out the features that would make little contribution to the response variable. If the data type of cell $DT_{nk}$ is binary, Fisher's exact test 324 may be used most effectively; otherwise, Wilcoxon rank-sum test 326 may be used. In both methods, features are selected when their $p$-values are smaller than 0.05. While Fisher's exact test 324 and Wilcoxon rank-sum test 326 are utilized in this exemplary embodiment, other tests may be used within the context of the present invention, including but not limited to: wrapper-based feature selection methods such as the use of predictive models to score feature subsets prior to selection, filter-based feature selection methods such as the use of mutual information or Pearson correlations, or embedded feature selection methods such as the least absolute shrinkage and selection operator (LASSO).

**4. Sample balancing:** The sample sizes of output classes are usually biased and imbalanced, especially in medical data. Consequently, the accuracy of the prediction result is often overestimated. In order to improve accuracy, optionally a sample balancing method is also applied. First, the major classes are randomly separated into many parts. Each part contains a sample size close to that of the minor class. Second, every part of the major class is combined with the minor class as training sets 332. The input data may be separated into several parts for cross validation, for example ten parts in the process of 10-fold cross validation: nine parts may then be taken to do sample balancing 336 and the remaining one as testing set 334 used to validate prediction models 340. Training sets 332 are balanced, while testing set 334 for validation is still imbalanced in the sample sizes of classes, providing a more reliable performance.

**5. Prediction models 340:** For comparisons, prediction models 340 optionally include two independent procedures: 1) machine learning - support vector machines (SVM), and 2) statistical modeling - logistic regression. A Support Vector Machine (SVM) software package may be used, for example a SVM package in the R programming language called "e1071". For kernel functions, a nonlinear function such as a Gaussian radial basis function may be used, which is also the soptimized kernel function. This SVM package provides fitted probabilities numerically from 0 to 1, and so does the logistic regression package used, named as "generalized linear models". The validity of predictive models 340 may be assessed in Performance Assessment 350. Software running on computer system 210 may thus serve as a toxicity module that determines if a toxicity reaction is likely based on an expanded set of targets to output the evaluation of the likelihood of toxicity for the particular drug with the particular patient.

**6. An example for predicating drug cardiotoxicity:** Here we use cardiotoxicity as an example to demonstrate how to apply our ADR prediction approach based on drug target-expanding network modeling. There are many ADRs related to cardiotoxicity, according to the index of the International Classification of Diseases 10th Revision (ICD-10). We merge all ADRs, each of which has an index ranging from 100 to 199 (classified as diseases of the circulatory system), into one group, $S_H$. The ADRs related to cardiotoxicity in SIDER and their ICD-10 indices are listed in Table 1. In the ADR vs. drug target/drug target expanding network facts (See the framework in FIG.1), if any one of $DS_{nh}$ is 1, where $D_n$ is drug n, and $S_h$ is in the group of heart-related ADR (see Table 1), then $DS_{nH}$ is set to 1; otherwise, $DS_{nH}$ is set to 0.

**TABLE 1**

| ADRs in SIDER | ICD-10 Index |
|---|---|
| Valvular Heart Disease | I08.8 |

(continued)

| ADRs in SIDER | ICD-10 Index |
|---|---|
| Rheumatic Carditis | I09.9 |
| Myocardial Infarction | I21 |
| Myocardial Ischemia | I25.6 |
| Heart Disease | I30-I52 |
| Constrictive Pericarditis | I31.1 |
| Pericardial Effusion | I31.3 |
| Cardiac Tamponade | I31.9 |
| Pericarditis | I32.8 |
| Endocarditis | I39.8 |
| Myocarditis | I40.8 |
| Cardiomyopathy | I42 |
| Second Degree Heart Block | I44.1 |
| Complete Heart Block | I44.2 |
| Heart Block | I45.5 |
| Cardiac Arrest | I46 |
| Sinus Tachycardia | I47 |
| Tachycardia | I47 |
| Junctional Tachycardia | I47.1 |
| Multifocal Atrial Tachycardia | I47.1 |
| Nodal Tachycardia | I47.1 |
| Supraventricular Tachycardia | I47.1 |
| Paroxysmal Ventricular Tachycardia | I47.2 |
| Ventricular Tachycardia | I47.2 |
| Heart Failure | I50 |
| Congestive Heart Failure | I50.0 |
| Right Heart Failure | I50.0 |
| Cardiomegaly | I51.7 |
| Cardiac Abnormality | I97.1 |

We evaluate the performance of ADR predictions in multiple experiments by applying standard statistical performance-evaluation measures, i.e., AUC (area under ROC curve), ACC (accuracy), SEN (sensitivity), and SPE (specificity). For each evaluation experiment, we repeat the experiments multiple times and report the statistical results, for example performing 10-fold cross validation three times and take median values to report prediction performances.

**1) Drug target-expanding PPI network modeling improves drug ADR predictions:** We evaluated drug ADR prediction performance by integrating different sets of confidence-ranked PPI data derived from the HAPPI database. The database contains comprehensive human functional and physical protein interaction/association data, at different confidence levels, from "1 Star" (low confidence, mostly functional association data) to "5 Star" (high confidence, mostly physical interaction data).

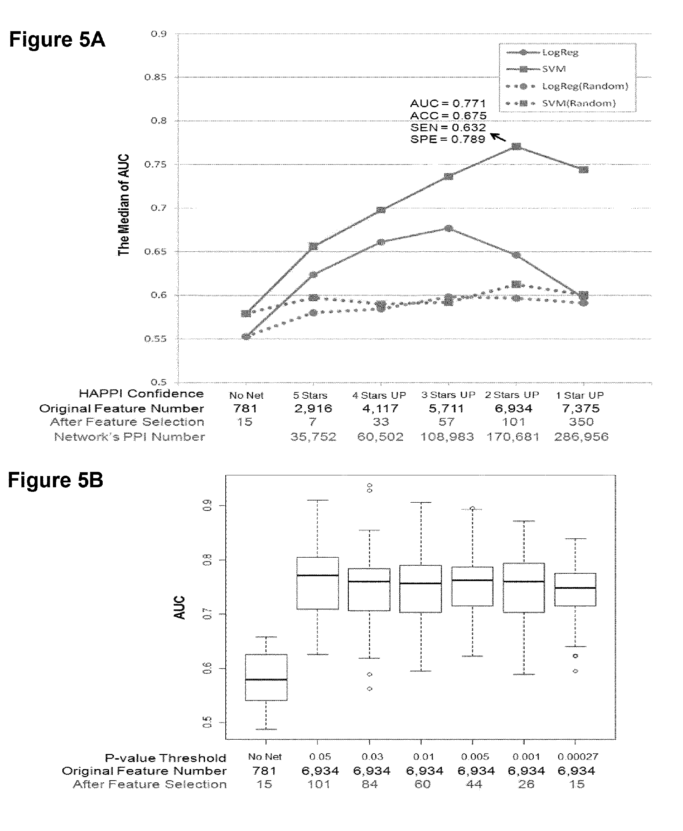

We can observe significant contributions of PPI networks to both prediction models, as shown in Figure 5A. When the SVM line is applied, the performance prediction goes up from AUC = 0.579 (using "No Net", or not PPI network data) to AUC = 0.771 (using "2 Stars UP" PPI network data). The use of PPI data brings up prediction performances significantly, i.e., Accuracy = 0.675, Sensitivity = 0.632, and Specificity = 0.789. The increased AUC of the "2 Stars UP" condition over the "No Net" condition is significant, withp-value = 4.93e-35 based on the t-test. By further including the lowest confidence level ("1-Star" PPI network data) into the drug target-expanding network, the prediction performance decreases slightly due to noise in molecular networks. The performance curve of the logistic regression line is comparable to, yet systematically lower than, that of SVM, moving up from AUC = 0.553 (using "No Net") to AUC = 0.677 (using "3 Stars UP" PPI network data). The performance of "3 Stars UP" PPI network data is lower than that of "2 Stars UP" PPI network data, at Accuracy = 0.649, Sensitivity = 0.564 and Specificity = 0.789. The increased AUC of the "3 Stars UP" condition over the "No Net" condition is also significant, withp-value = 6.83e-18 based on the t-test. However, the decreased AUC performance between "3 Stars UP" condition over the "2 Stars UP" condition is also noticeable, likely due to the functional nature (no longer biased towards physical PPI events) of biomolecular networks at the "2 Stars" level reported by the HAPPI database.

In order to control for the effects of using any types of (random) biomolecular networks and their possible contributions to ADR predictions, the model's performance was also evaluated with the use of randomized PPI networks which shared the same network topologies as actual PPI networks. Figure 5A also shows that the performance curves using random networks slightly increased (with AUC > 0.55), when the SVM line and logistic regression line were applied. This result occurs because the original relationships between drugs and drug targets are still retained in the simulated random PPI networks. The additional gained prediction power, however, may only be explained by the embedded useful network information that our prediction model automatically learned from real biological network structures. These results show that the contribution of PPI network data to drug ADR prediction is primarily due to useful functional information embedded in biomolecular functional association networks of drug targets and their related proteins, whereas network topology alone only plays a peripheral role.

We also assessed whether the increase in our model's prediction performance may be due to the increase in the total number of features when PPI network data are introduced. For this purpose, we focused on the result obtained from the use of "5 Stars" PPI network data, in which the number of features obtained by the prediction models becomes much smaller than that without using any network information. We noted that the AUC of this experimental result is better than that without using any network information ($p$-value = 2.70e-8 and 8.22e-9 for $T$-test, when we used SVM and logistic regression, respectively). To further confirm the relationship between the number of features captured in the model and the model performance, we performed another experiment in which we gradually decreased feature number "2 Stars UP" PPI data in the SVM prediction model by lowering feature selection thresholds. Figure 5B shows that there is no significant ($p$-value = 0.469 using ANOVA) decrease of prediction performances, when the number of features is filtered down. These observations further support our original finding that the contribution of PPI network for a drug's ADR prediction performance primarily comes from network data themselves.

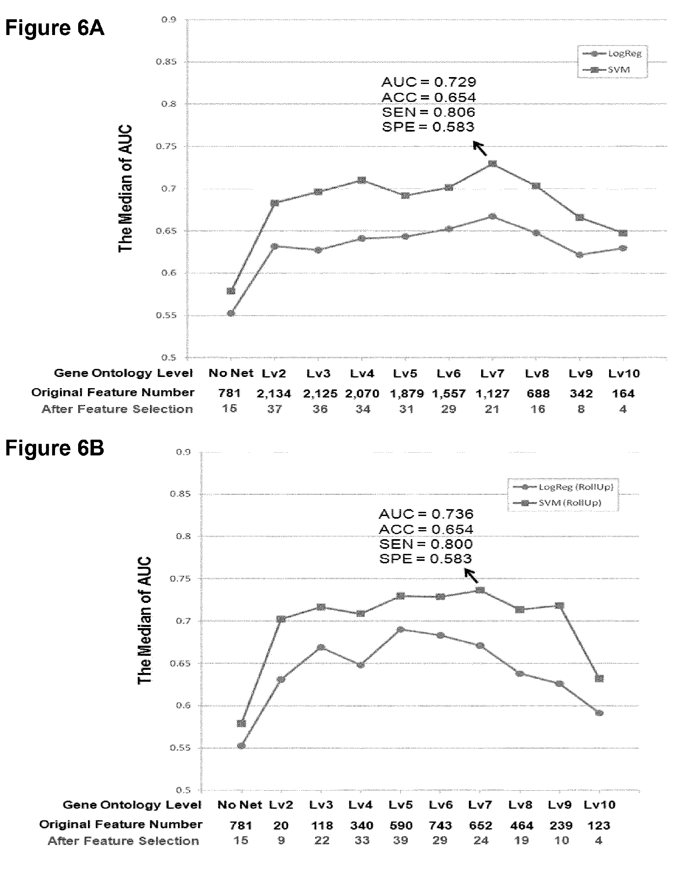

**2) Drug target-expanding GO network modeling also improves drug ADR predictions:** We evaluated drug ADR prediction performance by integrating GO annotations available for each drug's protein targets. In two experiments, shown in Figures 6A and 6B, we directly incorporated GO annotation labels of drug target proteins into our prediction models. Since each protein-coding gene may be annotated by many GO terms from different GO hierarchical levels, we carefully designed experiments to eliminate potential ADR prediction performance biases due to non-uniformity of GO term hierarchical levels. We aggregated GO terms to different GO hierarchical levels by applying different thresholds. Since GO hierarchical level=1 is not biologically meaningful and there is insufficient data for GO hierarchical levels from 11 to 15, results for these categories are not shown.

In Figure 6A, the GO terms equal to or deeper than specified threshold GO hierarchical levels are used to annotate drug targets for comparative drug ADR prediction performance analysis. Our results suggest that the prediction performances with the use of GO terms, regardless which predictive modeling method is used and which criteria is used for comparisons, are always better than those without the use of GO terms. In particular, when GO term level 7 (Lv7) is chosen, a best performance may be achieved with the use of SVM, in which we observed AUC = 0.729 and Sensitivity = 0.806; in comparison, "No Net" (without the use of GO term information) has AUC = 0.579. The improvement in overall ADR prediction performance defined by AUC is significant ($p$-value = 1.80e-18, based on $t$-test).

In Figure 6B, the GO terms deeper than level N are replaced by their level N GO term ancestors to annotate drug targets for comparative drug ADR prediction performance analysis. We call this process a "Roll Up" and observed similar results as in the first experiment. In particular, when GO term Lv7 is chosen, a best performance can be achieved with the use of SVM, in which we observed AUC = 0.736 and Sensitivity = 0.800. The improvement in overall ADR prediction performance defined by AUC over the "No Net" experiment is also determined to be statistically significant ($p$-value = 7.75e-17, based on i-test).

Based on the above two experiments using GO terms, we understand the following results. First, the use of GO annotations improves a drug's overall ADR prediction performance. Drug ADR prediction performances achieved with the best use of GO annotation (AUC = 0.736) are almost comparable to those achieved with the best use of PPI networks (AUC = 0.771). Second, SVM models achieve better performance than logistic regression models. Third, to achieve better ADR prediction performance, both SVM models and GO biological process use categorical terms at sufficiently detailed term levels (e.g., level 7) to annotate drug targets. Fourth, by evaluating detailed prediction performances achieved with PPI networks (SEN = 0.632, SPE = 0.789) and GO annotations (SEN = 0.800, SPE = 0.583), the integration of biomolecular network data increases the specificity (SPE) of ADR predictions, while the integration of GO annotation data increases the sensitivity (SEN) of ADR predictions.

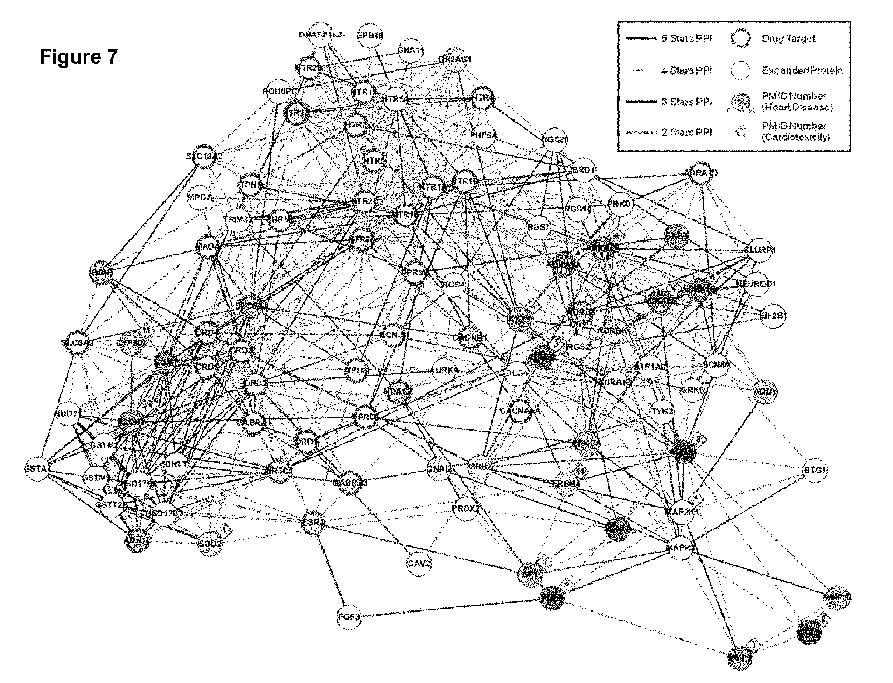

**3) A good ADR prediction model is concentrated not only on drug targets implicated with the ADR events, but also on many non-target proteins directly linked to ADR mechanisms:** We further investigated the biological network contexts for 101 proteins selected automatically by the SVM prediction model as features. We expanded these "seed proteins" with "2 Stars UP" PPI interactions to build a PPI interaction network shown in Figure 7, by using the nearest neighborhood expansion method. We used node color and counts (in diamond shapes) to show how much evidence from PubMed might be identified in each protein.

Many selected proteins are closely associated with cardiotoxicity. For example, ADRB1 (Adrenergic, beta-1-, receptor) mediates hormone epinephrine and neurotransmitter norepinephrine. The polymorphisms of ADRB1 have been shown to be involved in drug cardiotoxicity in heart failure. Autoantibodies against the beta-1-adrenergic receptor have also been shown to have idiopathic dilated cardiomyopathy in some patients. Therefore, ADRB1 as a known drug target and serves as a reliable predictor.

We also observed that the drug target-expanding network may bring forth additional cardiotoxicity-related non-target proteins, e.g., ERBB4 and CYP2D6. ERBB4, a v-erb-a erythroblastic leukemia viral oncogene homolog 4, is a member of the type I receptor tyrosine kinase subfamily and encodes a receptor for NDF/heregulin. Targeted deletion and inhibition of ERBB4 signaling may lead to congestive heart failure resulting from cardiovascular defects. CYP2D6 encodes a subunit of the cytochrome P450 superfamily of enzymes. The gene is specifically expressed in the right ventricle and its genetic polymorphism is known to be associated with cardiotoxicity, including a patient's poor anti-arrhythmic activity, severe cardiovascular, or dilated cardiomyopathy.

**Toxicity and Efficacy Analysis.** In post-genome biology, molecular connectivity maps have been proposed to establish comprehensive knowledge links between molecules of interest in a given biological context. Embodiments of the present invention use the computational connectivity maps (C-Maps) web server, which is an online bioinformatics resource that provides biologists with potential relationships between drugs and genes in specific disease contexts, to construct an integrative disease drug-perturbation pathway/network model. Alternatively, other connectivity maps may be used, based in whole or in part on information such as that of drug perturbed gene expression profiles, the Iconix database, and the Library of Integrated Network-based Cellular Signatures (LINCS) resources.

A pathway/network model-based drug ranking algorithm is developed to evaluate the pharmacological effects of drugs on the target proteins as either "therapeutic" or "toxic". A quantitative score for each drug can be calculated by determining the functional importance of targeted proteins, summarized pharmacological effects, and network topological features.

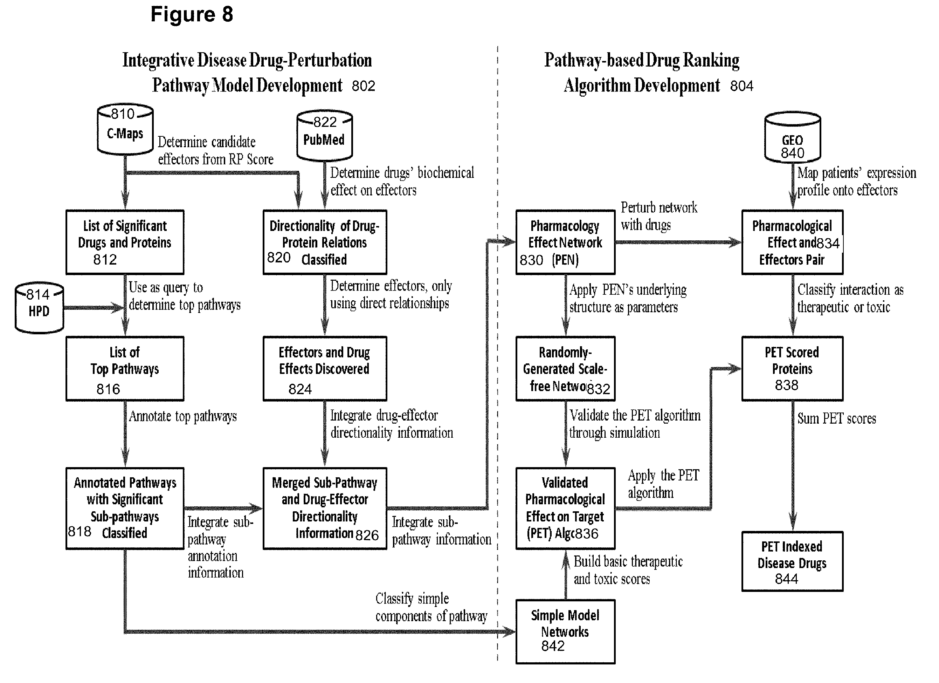

Our innovations open a new way to evaluate the efficacy of candidate drugs or drug combinations for patients with a specific disease. The framework includes two major parts as shown in Figure 8, the integrative disease drug-perturbation pathway model development process 802 and pathway-based drug ranking algorithm development process 804.

**1. Develop Integrative Disease Drug-Perturbation Pathway Models:** First, an integrated disease-specific pathway is constructed that consists of important disease-related drugs and proteins, through the C-Maps webserver 810, by simply inputting disease name. C-Maps 810 measures the importance of disease-related proteins by using RP score (for example, as may be calculated by the methodology disclosed in reference 2). After generating list 812 of important proteins for a disease, all the disease-related proteins are collectively used as a query search against the Human Pathway Database 814. This method yields comprehensive list 816 of important pathways related to the specific disease. The importance of each pathway is determined by how many proteins are included in the pathway. Each important pathway may then be annotated and, optionally, only sub-pathways with disease-related proteins are considered into list 818. Alternatively, one could use one or more these pathway or gene set data resources as substitutes for HPD 814, e.g., the Kyoto Encyclopedia of Genes and Genomes (KEGG) database by Kanehisa Laboratories, Kyoto University & University of Tokyo; the Reactome database (http://www.reactome.org) by the National Institutes of Health, Enfin of the European Union, and Ontario, Canada, New York University, and Cold Spring Harbor Laboratory; Curated Gene Signatures database (GeneSigDB at http://compbio.dfci.harvard.edu/genesigdb/), and the Pathway And Gene Enhanced Database (PAGED) database by Indiana University School of Informatics (http://bio.informatics.iupui.edu/paged/).

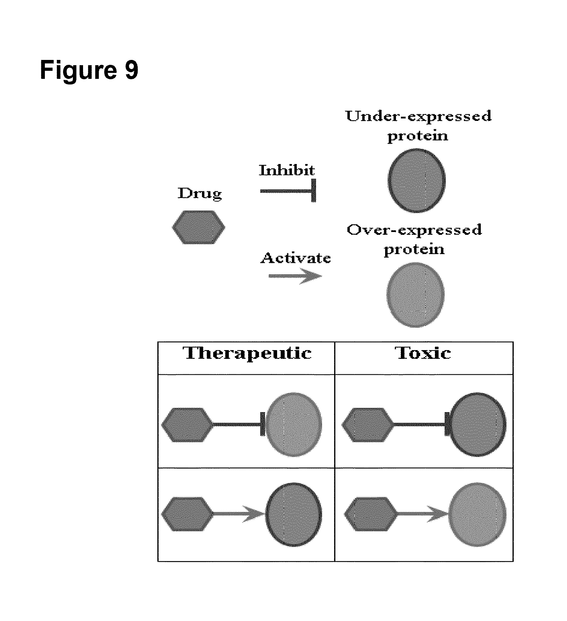

Second, directionality of protein-protein relations is obtained from the pathways that were annotated and mapped in list 820. Up-regulated relations are represented as pointed arrows and down-regulated relations are represented as flattened inhibitory arrows in Figure 9. Any top-ranked proteins from C-Maps 810 that are not included in any existing pathway are considered as "holes", which may be filled by using publicly available information about proteins, such as images and implementing the query term of Uniprot ID for the proteins combined with "Pathway". By examination of the pathway images retrieved, the holes of the pathway may be filled and thus generate an integrated pathway model with important disease-related proteins and the interactions (i.e. activation, inhibition, etc.) between them.

Third, drugs are mapped to the pathway based on either drug target information from DrugBank or the drug protein pairs from C-Maps 810. To determine how a specific drug affects a specific protein, we curate all the supported abstracts from PubMed 822 for each drug-protein pair from C-Maps 810, either manually or by an automated process. Each drug-protein relation is then classified as "up-regulated", "down-regulated", or "other". In step 824 a drug-protein relation is considered "up-regulated" whenever a drug positively influences a protein; "down-regulated" whenever a drug negatively influences a protein. These relations are then mapped as the edge attributes of the drug-protein interactions in 826. Alternatively, this curation of drug-protein relationships may be done with the use of a generally available natural language processing (NLP) software-based approach, or database lookup using a resource such as the Search Tool for Interactions of Chemicals (STITCH, athttp://stitch.embl.de/) database.

Finally, we translate the integrated disease-specific pathway model with mapped pharmacology effects - Pharmacology Effect Network 830 (PEN) - into a standard weighted network 832 by representing its components as an adjacency matrix and network perturbation vectors to facilitate the use of standard mathematical approaches.

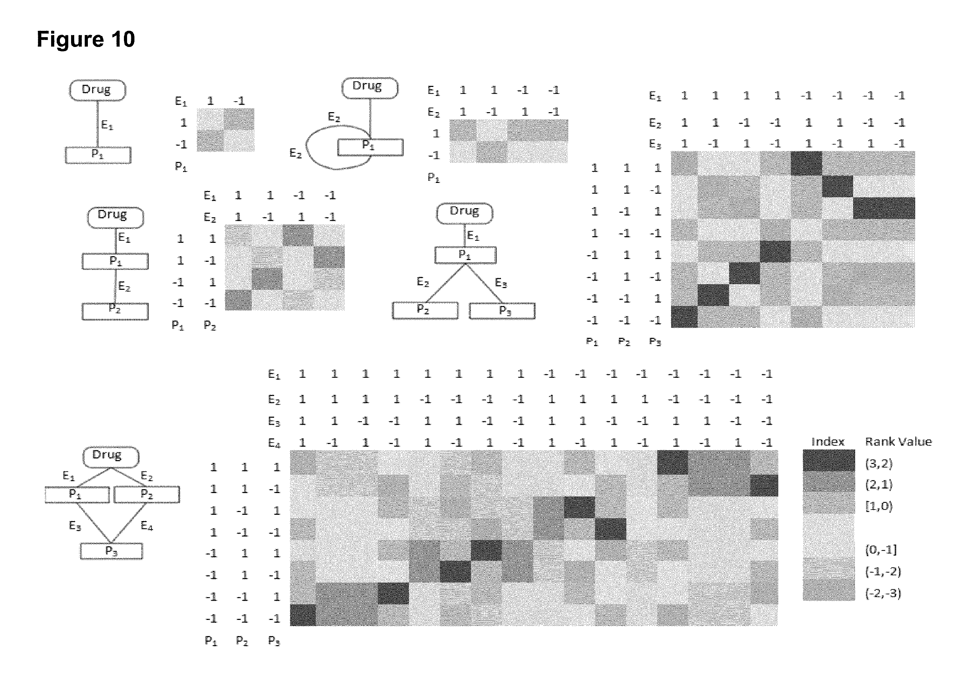

1) A standard weighted adjacency matrix, A (Figure 10), is used to store all the protein-protein interaction information from the PEN. Each protein or protein complex, which is represented as vertex in the network, is uniquely associated with a column-row pair in the adjacency matrix so that the *i*-th row and column contains the edge weight, which is the protein-protein interaction directionality. Therefore, the $a_{i,j}$ element contains the interaction between the protein associated with *i*-th row and *j*-th column of the matrix and is '1' if the proteins have a stimulating relationship, '-1' if the proteins have an inhibitory relationship, and '0' if no direct interaction exists between the proteins.

2) The drug-target interactions from the PEN are treated separately from the protein-protein interactions and stored in a perturbation vectors, $v_{d0}$, where *d* is the drug identifier. Each drug is also a vertex with drug-protein interactions represented as edge weights. Hence, maintaining the same unique row association for proteins and protein complexes as in A, the *i*-th row of the vector contains the interaction directionality between the drug and row associated protein using the same weighting outlined in 1).

**2. Develop PEN Model-based Drug Ranking Algorithms:** Ideal drugs for a patient diagnosed with a certain disease modulates the gene expression profile of this patient (from GEO 840) to the similar level with those in healthy individuals at pathway-level, as shown in Figure 9, we classify the drug's pharmacological effect on a protein into three categories in 834:

1) Therapeutic: if the drug activates the under-expressed protein or inhibits the over-expressed protein.

2) Toxic: if the drug activates the over-expressed protein or inhibits the under-expressed protein

3) Ambiguous: if there is missing directionality information for proteins or drugs.

The ranking algorithms assigns a high score for drugs that have a therapeutic effect on the proteins in the pathway and a low score for those that have a toxic effect on the proteins in the pathway. Since the ranking of a drug is essentially based on the Pharmacological Effect on its Targets, we call it a PET algorithm.

Since there may be multiple paths from the drug to its effected protein or effector and each one of these paths may have either a therapeutic or toxic effect, we developed an equation (1) to account for this fact. This equation is based on the information theory to score the overall effect of the specific drug to its effector.

$$w(N_m) = \frac{N_m}{N} log_2\left(\frac{2^k}{N}\right) \qquad (1)$$

Where $N_m$ is the number of the pharmacology effect of type m, where *m=1* for therapeutic and *m=2* for toxic. N is the total number of effects, and $2^k$ is a boosting factor based on the path length, *k,* from the drug to the effector. These two weights are combined to find the overall pharmacological effect of the drug to that effector using a logistic function to scale the results.

$$r_i = \frac{2}{1+e^{-(w(N_1)-w(N_2))}} - 1 \qquad (2)$$

Where $r_j$ can increase if the number of therapeutic affects increase and decrease if the numbers of toxic effects increase.

In order to obtain an accurate count for $N_1$ and $N_2$, a novel way to track the perturbations spread was developed. A drug can have multiple paths towards an effector and different paths may have conflicting effects, therefore an iterative approach was employed to separately classify each of the interactions involved as therapeutic or toxic. Therefore, each protein has an interaction vector associated with it, $i_{p,k}$, where k is the iteration index and p the proteins identifying number in the adjacency matrix

$$i_{p,k} = \begin{bmatrix} N_1 \\ N_2 \end{bmatrix} \qquad (3)$$

The initial perturbation is applied by updating the directly targeted effector proteins. The effects for the next step in the perturbation are found by storing the total number of outgoing and incoming paths for each node at a given step. In this way, we can systematically trace drugs' effects on the pathway across multiple discrete steps. In order to determine the effects of the incoming perturbation, the $v\overline{k+1}$ perturbation vector is calculated by multiplying the *p*-th column of *A, $^T$ $A^T(p, :)$,* by $v\overline{k}^T$.

$$\overrightarrow{v_{k+1}} , = A^T(p, :)\overrightarrow{v_k}^T \qquad (4)$$

By examining the *p*-th element of $v\overline{k+1}$, the corresponding proteins vector $i_{p,k+1}$ is vector is updated with all incoming path effects as follows.

$$i_{p,k+1} = i_{p,k} + \sum_{m(p)>0} (i_{n,k} - i_{p,k-1}) + \sum_{m(n)<0} inv(i_{n,k} - i_{n,k-1}) \qquad (5)$$

Where the *inv* function inverts the vector so that

$$inv(i_{p,k}) = \begin{bmatrix} N_2 \\ N_1 \end{bmatrix} \qquad (6)$$

All incoming paths from the previous protein receive opposite classifications for the next protein in the path due to a down-regulating edge.

The final normalized PET score which contains the information of the drug's overall effect on the whole pathway was found using the following equation:

$$r_{total} = \sum_{i=0}^{n} \frac{r_i}{\sum_i Rp(i)} \cdot \frac{n}{P} \qquad (7)$$

Where n is the total number of effector proteins in the integrated pathway, *m* is the total number of effector proteins in the *i*-th drugs sub pathway, and P is the total number of proteins in the sub network for the drug.

Where n is the total number of drug affected proteins in the integrated pathway. In order to compare multiple drugs effect on the same disease, a normalized PET index is employed to account for variance in the Rp score of the proteins in a drugs effector profile.

**3. Evaluate Breast Cancer Drug Efficacy by Using the PEN-PET Approach:** The top 500 drug-protein relations for breast cancer from C-Maps 810 against HPD 814 are searched to retrieve the top 15 pathways. All 15 pathways are annotated and integrated. The final integrated pathway contains a total of 221 nodes. Out of the 221 nodes, 188 nodes are for proteins in the pathway with those proteins from C-Maps 814 labeled as an oval while others encased in a rectangle; 23 nodes are for drugs and 14 nodes for biological processes such as "apoptosis" and "angiogenesis".

C-Maps 814 provide a comprehensive list of PubMed 822 abstracts for each of the 500 drug-protein relations for Breast Cancer. This amounts to over 5000 PubMed abstracts that were manually curated, classified, and categorized for breast cancer. After performing manual curation, 79 drug protein pairs contained only up information, 57 only down, 11 primarily up, 8 primarily down, and 345 unknowns.

A breast cancer PEN may be created with 23 drugs attached, including 6 FAD approved drugs for breast cancer. This network is then studied to determine compliance with the scale free property. Since PEN is a directed network, a degree distribution is generated for both in degree and out degree. Both of them show a scale free property with an R square of 0.95 for in degree and 0.96 for out degree.

PET algorithm on simple networks was tested, which are the functional building blocks of large, complex networks, such as the one drug with one target proteins, simple loop, and so on. Such testing makes sure that PET is logically correct for those simple networks. Figure 10 shows one of the simple networks tested.

Note that the main diagonal has toxic effects due to the drug's pharmacological effect on its protein and the protein expressions in these cases having the same signs (over-expressed and activating, under expressed and inhibiting, etc.). Similarly the opposite diagonal has opposite effect types and receives largely therapeutic scores.

Then we applied PET algorithm on two breast cancer related gene expression microarray datasets (GSE3193, GSE10886), in which samples from breast cancer patients are all Estrogen Receptor positive (ER+). Differential expressed genes were mapped onto the PEN to create a disease specific network.

Of the 23 drugs tested, 3 received purely positive scores, including two popular breast cancer treatments specifically for ER+ patients, Tamoxifen and Raloxifene; 11 received purely negative PET scores, including one drug withdrawn from the market, nine drugs either the lack of any clinical trials or as with few clinical trials. Mitomycin is the only breast cancer treatment to receive a negative score across both expression profiles. As Mitomycin is an older chemotherapy drug targeting at DNA rather than specific proteins, this result was anticipated. The breast cancer drugs Exemstane, Anastrozole and Letrozole all target at CYP19A1 and thus received the same scores. Due to a mixture of therapeutic and toxic effects on effector proteins in ER+ gene expression profiles, their individual PET scores for the proteins are quite low.

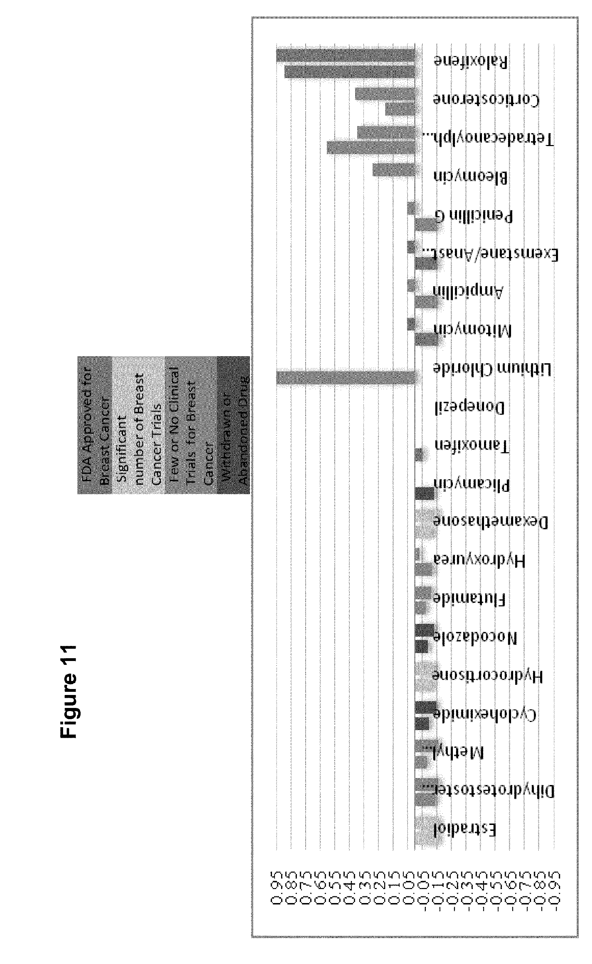

In sum, Figure 11 shows a trend towards ranking withdrawn or abandoned drugs with a negative score while FDA approved drugs rank with a positive score; within the FDA drugs, scores favor those drugs specifically for ER+ patients since both microarray datasets are from ER+ patients.

The significance of manually curating a drugs effect on breast cancer proteins is highlighted in the case of Ralxofiene. Raloxifene targets a key breast cancer protein ESR1. It was predicted that Raloxifene would receive a highly positive score. However, contradictory information exists about the effects of this drug on ESR1. For example, in breast tissue Raloxifene inhibits ESR1, causing a therapeutic effect, but in bones, Raloxifene can activate ESR1, which would be classified as toxic. Therefore, the use of manual curation to determine the effects on ESR1 is significant so that the appropriate interaction may be selected. In Figure 11 Raloxifene* indicates the score that Raloxifene would receive if the wrong effect had been selected.

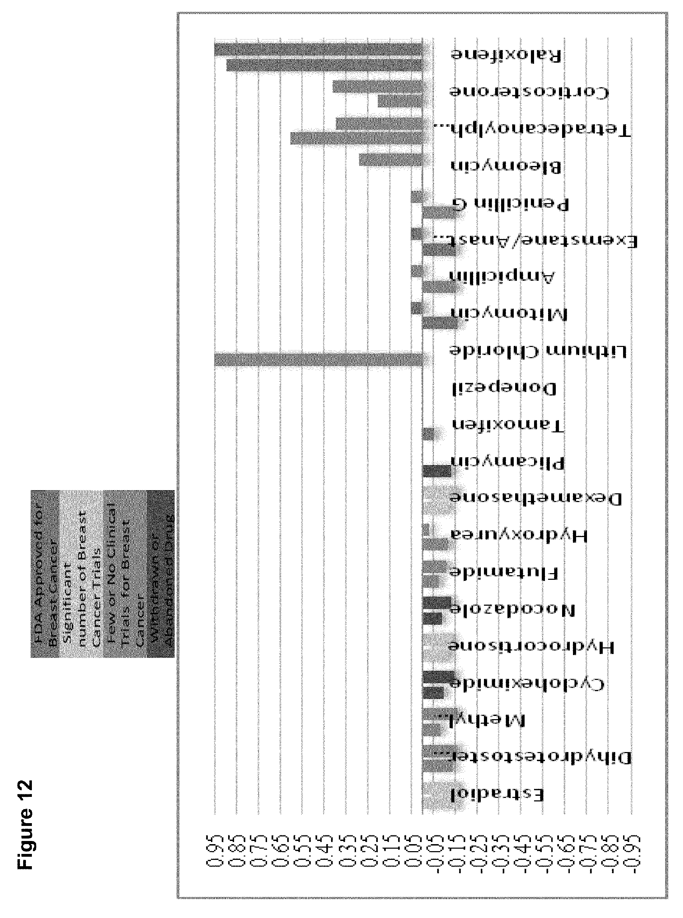

An alternate approach to rank drug's efficacy is to ignore the underlying network structure and only examine the ultimate effect of the drug on the effector proteins. This approach has previously been used. In this approach the effect on each protein is classified as either entirely toxic or entirely therapeutic. This would be the equivalent of taking only the sign of the PET score. However, as Figure 12 demonstrates, this algorithm has trouble differentiating between drugs with therapeutic outcomes and those without.

The following references were used in the development of the present invention, and the disclosures of which are explicitly incorporated by reference herein:

1. Knox, C., et al., DrugBank 3. 0: a comprehensive resource for 'omics' research on drugs. Nucleic Acids Res, 2011. 39(Database issue): p. D1035-41.

2. Kuhn, M., et al., A side effect resource to capture phenotypic effects of drugs. Mol Syst Biol, 2010. 6: p. 343.

3. Chen, J.Y., S. Mamidipalli, and T. Huan, HAPPI: an online database of comprehensive human annotated and predicted protein interactions. BMC Genomics, 2009. 10 Suppl 1: p. S16.

4. Ashburner, M., et al., Gene ontology: tool for the unification of biology. The Gene Ontology Consortium. Nat Genet, 2000. 25(1): p. 25-9.

5. Hornik, K., The R FAQ. 2011.

6. Oommen, T., Sampling Bias and Class Imbalance in Maximum-likelihood Logistic Regression. Math Geosci, 2011. 43: p. 99-120.

7. Meyer, Support Vector Machines: The interface to libsvm in Package e1071. 2004.

8. Geyer, C.J., Generalized Linear Models in R. 2003.

9. Geneva, The ICD-10 classification of mental and behavioural disorders: clinical descriptions and diagnostic guidelines. World Health Organization, 1992.

10. Chen, J.Y., C. Shen, and A.Y. Sivachenko, Mining Alzheimer disease relevant proteins from integrated protein interactome data. Pac Symp Biocomput, 2006: p. 367-78.

11. Ranade, K., et al., A polymorphism in the beta1 adrenergic receptor is associated with resting heart rate. Am J Hum Genet, 2002. 70(4): p. 935-42.

12. Magnusson, Y., et al., Mapping of a functional autoimmune epitope on the beta 1-adrenergic receptor in patients with idiopathic dilated cardiomyopathy. J Clin Invest, 1990. 86(5): p. 1658-63.

13. Bernstein, D., et al., Differential cardioprotective/cardiotoxic effects mediated by beta-adrenergic receptor subtypes. Am J Physiol Heart Circ Physiol, 2005. 289(6): p. H2441-9.

14. Christ, T., et al., Autoantibodies against the beta1 adrenoceptor from patients with dilated cardiomyopathy prolong action potential duration and enhance contractility in isolated cardiomyocytes. J Mol Cell Cardiol, 2001. 33(8): p. 1515-25.

15. Fuller, S.J., K. Sivarajah, and P.H. Sugden, ErbB receptors, their ligands, and the consequences of their activation and inhibition in the myocardium. J Mol Cell Cardiol, 2008. 44(5): p. 831-54.

16. Horie, T., et al., Acute doxorubicin cardiotoxicity is associated with miR-146a-induced inhibition of the neuregulin-ErbB pathway. Cardiovasc Res, 2010. 87(4): p. 656-64.

17. Thum, T. and J. Borlak, Gene expression in distinct regions of the heart. Lancet, 2000. 355(9208): p. 979-83.

18. Ovaska, H., et al., Propafenone poisoning--a case report with plasma propafenone concentrations. J Med Toxicol, 2010. 6(1): p. 37-40.

19. Lamb, J., et al., The Connectivity Map: using gene-expression signatures to connect small molecules, genes, and disease. Science, 2006. 313(5795): p. 1929.

20. Li, J., X. Zhu, and J.Y. Chen, Building disease-specific drug-protein connectivity maps from molecular interaction networks and PubMed abstracts. PLoS Comput Biol, 2009. 5(7): p. e1000450.

21. Chowbina, S.R., et al., HPD: an online integrated human pathway database enabling systems biology studies. BMC Bioinformatics, 2009. 10 Suppl 11: p. S5.

22. Hui Huang, X.W., Shuyu Li, Sara Ibrahim, Taiwo Ajumobi, and Jake Y. Chen, Evaluate Drug Effects on Gene Expression Profiles with Connectivity Maps, in 2nd International Workshop on Data Mining for Biomarker Discovery. 2010.

**Claims**

1. A toxicity analysis tool comprising a patient analysis module including a microarray and associated computer system configured to obtain gene expression information about a particular patient; **characterized by** a database module of said computer system configured to provide a set of targets for known interactions of a particular drug; a network interaction module of said computer system configured to expand said set of targets based on network interaction information to produce an expanded set of targets; and a toxicity module of said computer system configured to determine if a toxicity reaction is likely based on said expanded set of targets, said toxicity module outputting an evaluation of the likelihood of toxicity for the particular drug with the particular patient.

2. The toxicity analysis tool according to claim 1 **characterized in that** said patient analysis module is also configured to obtain at least one of RNA, DNA, protein, and metabolite information.

3. The toxicity analysis tool according to claim 1 or 2 **characterized in that** said database module includes at least one of drug, drug target information, and drug side effect information.

4. The toxicity analysis tool according to any of the preceding claims **characterized in that** said network interaction module uses at least one of a protein-protein interaction network model, gene ontology information including hierarchical terms, biological processes, cellular components, and molecular functions.

5. The toxicity analysis tool according to any of the preceding claims **characterized in that** said extended set of targets includes feature information associated with each target, and said tool further including a feature selection module configured to remove elements of said extended set of targets based on said feature information.

6. The toxicity analysis tool according to any of the preceding claims **characterized in that** said feature selection module is configured to filter said extended set of targets based on associated feature information having a p-value under a predetermined value, the predetermined value preferably being about 0.05.

7. The toxicity analysis tool according to any of the preceding claims **characterized by** a cross-validation module configured to balance said extended set of targets by partitioning said extended set of targets into a plurality of training sets and a testing set, and said cross-validation module balances said plurality of training sets.

8. The toxicity analysis tool according to any of the preceding claims **characterized by** a disease profile module configured to generate a list of of proteins related to the particular disease, select a plurality of drug pathways from a pathway database based on the list of proteins, provide an interface for annotating each of the plurality of drug pathways, map each drug-protein interaction on each of the plurality of drug pathways including identifying effector proteins, and translate the mapped plurality of drug pathways into a weighted network; a patient expression profile module configured to obtain a mapping of the gene-expression profile of the particular patient onto the effectors; and an evaluation module configured to calculate a ranking of the drugs associated with the plurality of drug pathways based on the effector proteins in each of the plurality of drug pathways and the mapping of the gene-expression profile of the particular patient, said evaluation module configured to provide a ranking of drugs associated with the pathways for treatment of the particular disease.

9. A method of determining toxicity of a drug for a particular patient including the steps of obtaining gene expression information and/or metabolite information about a particular patient using a microarray to analyze a patient sample, **characterized by** accessing at least one database and extracting a set of targets for known interactions of a particular drug; expanding the set of targets based on network interaction information to produce an expanded set of targets; and determining if a toxicity reaction is likely for the particular patient based on said expanded set of targets, said determining step including outputting an evaluation of the likelihood of toxicity for the particular drug.

10. The toxicity determination method according to claim 9 **characterized in that** said accessing step includes accessing at least one of drug, drug target information, and drug side effect information.

11. The toxicity determination method according to claim 9 or 10 **characterized in that** said expanding step uses at least one of a protein-protein interaction network model or gene ontology information including hierarchical terms, biological processes, cellular components, and molecular functions.

12. The toxicity determination method according to any of the preceding claims 9-11 **characterized in that** the extended set of targets includes feature information associated with each target, further **characterized by** removing elements of the extended set of targets based on feature information.

13. The toxicity determination method according to any of the preceding claims 9-12 **characterized in that** said removing step i ncludes filtering the extended set of targets based on associated feature information having a p-value under a predetermined value, the predetermined value preferably being about 0.05.

14. The toxicity determination method according to any of the preceding claims 9-13 **characterized by** the step of cross-validation by balancing the extended set of targets and partitioning the extended set of targets into a plurality of training sets and a testing set, then balancing said plurality of training sets.

15. The toxicity determination method according to any of the preceding claims 9-14 **characterized by** generating a list of of proteins related to the particular disease; selecting a plurality of drug pathways from a pathway database based on the list of proteins; annotating each of the plurality of drug pathways; mapping each drug-protein interaction on each of the plurality of drug pathways including identifying effector proteins; translating the mapped plurality of drug pathways into a weighted network; and calculating a ranking of the drugs associated with the plurality of drug pathways based on the effector proteins in each of the plurality of drug pathways and providing a ranking of drugs associated with the pathways for treatment of the particular disease.

FIG.1

FIG.2

EP 2 600 269 A2

Figure 3

**Figure 4A**

| Drug | $S_1$ | $S_2$ | ... | $S_J$ | $T_1$ | $T_2$ | ... | $T_K$ |
|------|-------|-------|-----|-------|-------|-------|-----|-------|
| $D_1$ | $DS_{11}$ | $DS_{12}$ | ... | $DS_{1J}$ | $DT_{11}$ | $DT_{12}$ | ... | $DT_{1K}$ |
| $D_2$ | $DS_{21}$ | $DS_{22}$ | ... | $DS_{2J}$ | $DT_{21}$ | $DT_{22}$ | ... | $DT_{2K}$ |
| ... | ... | ... | ... | ... | ... | ... | ... | ... |
| $D_N$ | $DS_{N1}$ | $DS_{N2}$ | ... | $DS_{NJ}$ | $DT_{N1}$ | $DT_{N2}$ | ... | $DT_{NK}$ |

**Figure 4B**

Drug Target Expanding Network

**Figure 4C**

Illustration of Expanding

## Figure 5A

| HAPPI Confidence | No Net | 5 Stars | 4 Stars UP | 3 Stars UP | 2 Stars UP | 1 Star UP |
|---|---|---|---|---|---|---|
| Original Feature Number | 781 | 2,916 | 4,117 | 5,711 | 6,934 | 7,375 |
| After Feature Selection | 15 | 7 | 33 | 57 | 101 | 350 |
| Network's PPI Number | | 35,752 | 60,502 | 108,983 | 170,681 | 286,956 |

## Figure 5B

| P-value Threshold | No Net | 0.05 | 0.03 | 0.01 | 0.005 | 0.001 | 0.00027 |
|---|---|---|---|---|---|---|---|
| Original Feature Number | 781 | 6,934 | 6,934 | 6,934 | 6,934 | 6,934 | 6,934 |
| After Feature Selection | 15 | 101 | 84 | 60 | 44 | 26 | 15 |

## Figure 6A

## Figure 6B

Figure 7

# Figure 8

## Integrative Disease Drug-Perturbation Pathway Model Development 802

## Pathway-based Drug Ranking Algorithm Development 804

**810 C-Maps**

Determine candidate effectors from RP Score

**822 PubMed**

Determine drugs' biochemical effect on effectors

**GEO 840**

Map patients' expression profile onto effectors

**List of Significant Drugs and Proteins 812**

**Directionality of Drug-Protein Relations 820 Classified**

Perturb network with drugs

**Pharmacology Effect Network 830 (PEN)**

**Pharmacological Effect and 834 Effectors Pair**

**814 HPD**

Use as query to determine top pathways

Determine effectors, only using direct relationships

Apply PEN's underlying structure as parameters

Classify interaction as therapeutic or toxic

**List of Top Pathways 816**

**Effectors and Drug Effects Discovered 824**

**Randomly-Generated Scale-free Netwo 832**

**PET Scored Proteins 838**

Annotate top pathways

Integrate drug-effector directionality information

Validate the PET algorithm through simulation

Sum PET scores

**Annotated Pathways with Significant Sub-pathways 818 Classified**

Integrate sub-pathway annotation information

**Merged Sub-Pathway and Drug-Effector Directionality Information 826**

Integrate sub-pathway information

**Validated Pharmacological Effect on Target (PET) Algc 836**

Apply the PET algorithm

Build basic therapeutic and toxic scores

**PET Indexed Disease Drugs 844**

Classify simple components of pathway

**Simple Model Networks 842**

# Figure 9

Figure 10

**Figure 11**

Legend:
- FDA Approved for Breast Cancer
- Significant number of Breast Cancer Trials
- Few or No Clinical Trials for Breast Cancer
- Withdrawn or Abandoned Drug

Drugs: Estradiol, Dihydrotestoster..., Methyl..., Cycloheximide, Hydrocortisone, Mocodazole, Flutamide, Hydroxyurea, Dexamethasone, Plicamycin, Tamoxifen, Donepezil, Lithium Chloride, Mitomycin, Ampicillin, Exemstane/Anast..., Penicillin G, Bleomycin, Tetradecanoylph..., Corticosterone, Raloxifene

**Figure 12**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **KNOX, C. et al.** DrugBank 3. 0: a comprehensive resource for 'omics' research on drugs. *Nucleic Acids Res,* 2011, vol. 39, D1035-41
- **KUHN, M. et al.** A side effect resource to capture phenotypic effects of drugs. *Mol Syst Biol,* 2010, vol. 6, 343
- **CHEN, J.Y. ; S. MAMIDIPALLI ; T. HUAN.** HAPPI: an online database of comprehensive human annotated and predicted protein interactions. *BMC Genomics,* 2009, vol. 10 (1), S16
- **ASHBURNER, M. et al.** Gene ontology: tool for the unification of biology. The Gene Ontology Consortium. *Nat Genet,* 2000, vol. 25 (1), 25-9
- **HORNIK, K.** *The R FAQ,* 2011
- **OOMMEN, T.** Sampling Bias and Class Imbalance in Maximum-likelihood Logistic Regression. *Math Geosci,* 2011, vol. 43, 99-120
- **MEYER.** *Support Vector Machines: The interface to libsvm in Package,* 2004, e1071
- **GEYER, C.J.** *Generalized Linear Models in R.,* 2003
- **GENEVA.** The ICD-10 classification of mental and behavioural disorders: clinical descriptions and diagnostic guidelines. World Health Organization, 1992
- **CHEN, J.Y. ; C. SHEN ; A.Y. SIVACHENKO.** Mining Alzheimer disease relevant proteins from integrated protein interactome data. *Pac Symp Biocomput,* 2006, 367-78
- **RANADE, K. et al.** A polymorphism in the beta1 adrenergic receptor is associated with resting heart rate. *Am J Hum Genet,* 2002, vol. 70 (4), 935-42
- **MAGNUSSON, Y. et al.** Mapping of a functional autoimmune epitope on the beta 1-adrenergic receptor in patients with idiopathic dilated cardiomyopathy. *J Clin Invest,* 1990, vol. 86 (5), 1658-63
- **BERNSTEIN, D. et al.** Differential cardioprotective/cardiotoxic effects mediated by beta-adrenergic receptor subtypes. *Am J Physiol Heart Circ Physiol,* 2005, vol. 289 (6), H2441-9
- **CHRIST, T. et al.** Autoantibodies against the beta1 adrenoceptor from patients with dilated cardiomyopathy prolong action potential duration and enhance contractility in isolated cardiomyocytes. *J Mol Cell Cardiol,* 2001, vol. 33 (8), 1515-25
- **FULLER, S.J. ; K. SIVARAJAH.** P.H. Sugden, ErbB receptors, their ligands, and the consequences of their activation and inhibition in the myocardium. *J Mol Cell Cardiol,* 2008, vol. 44 (5), 831-54
- **HORIE, T. et al.** Acute doxorubicin cardiotoxicity is associated with miR-146a-induced inhibition of the neuregulin-ErbB pathway. *Cardiovasc Res,* 2010, vol. 87 (4), 656-64
- **THUM, T. ; J. BORLAK.** Gene expression in distinct regions of the heart. *Lancet,* 2000, vol. 355 (9208), 979-83
- **OVASKA, H. et al.** Propafenone poisoning--a case report with plasma propafenone concentrations. *J Med Toxicol,* 2010, vol. 6 (1), 37-40
- **LAMB, J. et al.** The Connectivity Map: using gene-expression signatures to connect small molecules, genes, and disease. *Science,* 2006, vol. 313 (5795), 1929
- **LI, J. ; X. ZHU ; J.Y. CHEN.** Building disease-specific drug-protein connectivity maps from molecular interaction networks and PubMed abstracts. *PLoS Comput Biol,* 2009, vol. 5 (7), e1000450
- **CHOWBINA, S.R. et al.** HPD: an online integrated human pathway database enabling systems biology studies. *BMC Bioinformatics,* 2009, vol. 10 (11), S5
- **HUI HUANG, X.W. ; SHUYU LI ; SARA IBRAHIM ; TAIWO AJUMOBI ; JAKE Y. CHEN.** Evaluate Drug Effects on Gene Expression Profiles with Connectivity Maps. *2nd International Workshop on Data Mining for Biomarker Discovery,* 2010